# EUROPEAN PATENT APPLICATION

(11) **EP 1 541 997 A1**
(43) Date of publication of application: **15.06.2005**
(21) Application number: 04078339.1
(22) Date of filing: 09.12.2004
(51) Int. Cl.: G01N 27/12, G01N 33/00

(54) **Method and system for impedance measurement of a zeolite-based ammonia sensor**

(30) Priority: 12.12.2003 US 529368 P; 15.10.2004 US 966910
(71) Applicant: Delphi Technologies, Inc., Troy, MI 48007 (US)
(72) Inventor: Mowery, Kenneth D., Noblesville, IN 46060 (US); Tackitt, Douglas J., Kokomo, IN 46902 (US)
(74) Representative: Denton, Michael John

(57) **Abstract**

A method for measuring an impedance of a sensor (S1) that is subject to ion migration includes a number of steps. Initially, a first electrical pulse (VCELL) is applied to an input of the sensor (S1), whose impedance varies according to a first gas concentration in a gas stream. Next, a second electrical pulse (VCELL) is applied to the input of the sensor (S1). The energy of the first and second electrical pulses (VCELL) is approximately the same and the first and second electrical pulses (VCELL) have opposite polarity. A sensor load current (ICELL) is determined during at least one of the first and second electrical pulses (VCELL) to provide a first sensor current (ICELL). Then, the sensor load current (ICELL) during the same one of the first and second electrical pulses (VCELL) is determined to provide a second sensor current (ICELL). Finally, at least one component of the impedance of the sensor (S1) is determined based upon the first and second sensor currents (ICELL).

## Description

### Technical Field

The present invention is generally directed to measuring the impedance of a sensor and, more specifically, to measuring the impedance of a zeolite-based ammonia sensor.

### Background of the Invention

Various sensors have been developed to detect chemical elements and/or chemical compounds in a gas stream. For example, one zeolite-based sensor exhibits a complex impedance that is dependent on the concentration of ammonia (NH3) in a gas stream presented to the sensor. In one particular implementation, a zeolite-based sensor has been positioned within an exhaust gas stream of a diesel engine to provide feedback, as to the concentration of ammonia in the exhaust gas stream, to a control unit. Based upon the concentration of the ammonia in the exhaust gas stream, the control unit may cause reduction of the injection of urea, which acts to reduce nitrogen oxide (NOx) emission levels from the diesel engine, into the exhaust gas steam.

In many applications, in determining an impedance of a sensor it is undesirable to bias the sensor with a signal that has a direct current (DC) component, as the DC component may cause ion migration or other chemical reactions in the sensor. Ion migration in a sensor may alter the impedance of the sensor, thereby providing an incorrect indication of the level of a gas within a gas stream. A traditional approach for determining the impedance of a sensor has utilized a system that has sourced a sinewave voltage excitation to an input of the sensor and has observed the resulting sinusoidal current. In general, such systems have captured both the amplitude and phase relationship of the sensor voltage and the sensor current, such that both a real and imaginary part of a sensor impedance could be determined. However, it should be appreciated that a system designed according to the traditional approach can be relatively expensive and may experience difficulty in providing a desired accuracy over an extended period of time.

At least one zirconia-based wide range air fuel (WRAF) sensor has been designed to provide an impedance that can be correlated with a temperature of the sensor. In systems implementing a WRAF sensor that provides an impedance that can be correlated with the temperature of the WRAF sensor, the impedance has been determined through a number of techniques. One technique for determining an impedance of a WRAF sensor has been to periodically apply two successive pulses that have opposite polarity. In this technique, two current readings are taken during a first electrical pulse to obtain information that can be utilized to determine both reactive and resistive components of the impedance. Another technique for determining an impedance of a WRAF sensor has been to periodically couple a known resistive load between an input of the sensor and ground and measure a resultant voltage across the resistive load. However, neither of these techniques have generally been extended beyond zirconia-based WRAF sensors.

What is needed is a less expensive, less error prone technique for determining the impedance of a sensor, e.g., a zeolite-based ammonia sensor, that may experience ion migration.

### Summary of the Invention

One embodiment of the present invention is directed to a technique for measuring the impedance of a sensor that is subject to ion migration. Initially, a first electrical pulse is applied to an input of the sensor, whose impedance varies according to a first gas concentration in a gas stream. Next, a second electrical pulse is applied to the input of the sensor. The energy of the first and second electrical pulses is approximately the same, e.g., within +/- five percent, and the first and second electrical pulses have opposite polarity. Next, a sensor load current is determined during at least one of the first and second electrical pulses to provide a first sensor current. Then, the sensor load current during the same one of the first and second electrical pulses is determined to provide a second sensor current. Finally, at least one component of the impedance of the sensor is determined based upon the first and second sensor currents.

According to another embodiment of the present invention, a technique for measuring the impedance of a sensor subject to ion migration includes a number of steps. Initially, a first electrical pulse is applied to an input of the sensor, whose impedance varies according to a first gas concentration in a gas stream. Next, a sensor load current is determined during the first electrical pulse to provide a sensor current. Then, at least one component of the impedance of a sensor is determined based upon the first sensor current.

According to another embodiment of the present invention, the first sensor current is determined during the first electrical pulse at a first time and the second sensor current is determined during the first electrical pulse at a second time that occurs after the first time. According to still another embodiment of the present invention, the first sensor current is determined during the first electrical pulse, while the sensor load current is slewing, and the second sensor current is determined during the first electrical pulse when the sensor load current has approximately reached a steady-state value, e.g., within +/- five percent of the steady-state value.

According to still another embodiment of the present invention, the gas stream is an exhaust gas stream associated with a diesel engine. According to another aspect of the present invention, at least one component of the impedance is a resistive component. According to still another aspect of the present invention, the at least one component of the impedance includes a reactive component and a resistive component. According to another aspect of the present invention, the sensor is a zeolite-based sensor. According to a different embodiment of the present invention, the first gas concentration is an ammonia concentration and the gas stream is an exhaust gas stream associated with a diesel engine.

These and other features, advantages and objects of the present invention will be further understood and appreciated by those skilled in the art by reference to the following specification, claims and appended drawings.

### Brief Description of the Drawings

The present invention will now be described, by way of example, with reference to the accompanying drawings, in which:
Fig. 1 is a diagram of an exemplary system for removing nitrogen oxide (NOx) from an exhaust stream of a diesel engine;
Fig. 2 is a chart depicting impedance curves for a sensor at different ammonia (NH3) concentrations;
Fig. 3 is an electrical schematic of a circuit for providing pulses with opposite polarity to a sensor for determining at least one sensor impedance component, according to one embodiment of the present invention;
Fig. 3A depicts exemplary signals associated with the circuit of Fig. 3;
Fig. 4 is an electrical diagram of a circuit for providing pulses with opposite polarity to a sensor for determining at least one sensor impedance component, according to another embodiment of the present invention;
Fig. 4A depicts exemplary signals associated with the circuit of Fig. 4;
Fig. 5 is an electrical schematic of another circuit for applying pulses of opposite polarity to a sensor for determining at least one sensor impedance component, according to a different aspect of the present invention; and
Fig. 5A depicts exemplary signals associated with the circuit of Fig. 5.

### Description of the Preferred Embodiments

Various embodiment of the present invention are directed to techniques for measuring an impedance of a sensor that is subject to ion migration. It should be appreciated that the specific technique implemented will depend upon the type of sensor. For example, in sensors that are very sensitive to charge imbalance it can be advantageous to apply a first electrical pulse to an input of the sensor followed by application of a second electrical pulse to balance the charge on the sensor. According to one embodiment of the present invention, a first electrical pulse is provided to an input of a sensor whose impedance varies according to a first gas concentration in a gas stream. Next, a second electrical pulse is applied to the input of the sensor.

For charge balance, it is desirable for the energy of the first and second electrical pulses to be approximately the same, e.g., within +/- five percent of each other, and for the first and second electrical pulses to have opposite polarities. Then, a sensor load current is determined during at least one of the first and second electrical pulses to provide a first sensor current. Next, the sensor load current is determined during the same one of the first and second electrical pulses to provide a second sensor current. Finally, at least one component of the impedance of the sensor is determined based upon the first and second sensor currents.

According to another embodiment of the present invention, a technique for measuring the impedance of a sensor subject to ion migration includes the application of only a single electrical pulse to the sensor. It should be appreciated that this technique may not be satisfactory for sensors that have relatively long recovery times. According to this embodiment, a first electrical pulse is applied to an input of the sensor, whose impedance varies according to a first gas concentration in a gas stream. Next, a sensor load current is determined during the first electrical pulse to provide a sensor current. Then, at least one component of the impedance of a sensor is determined based upon the sensor current.

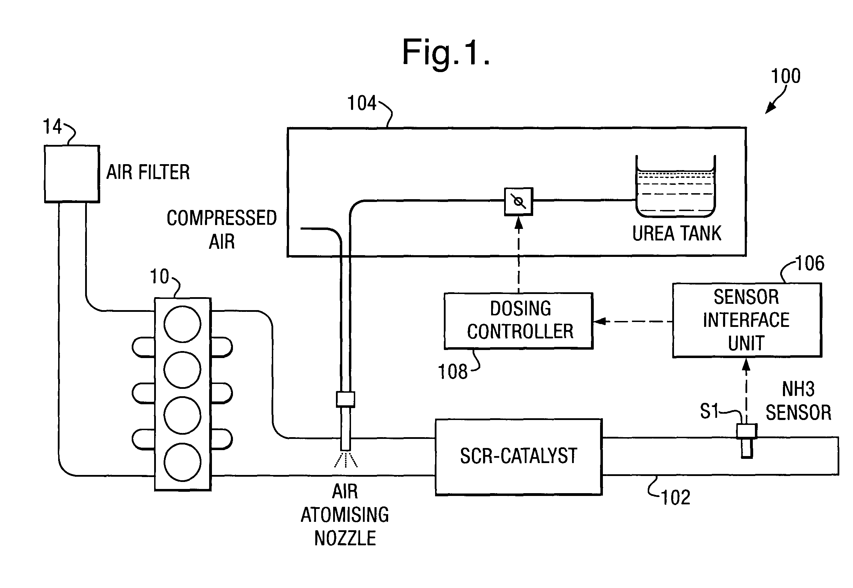

Fig. 1 depicts an exemplary system 100, which removes nitrogen oxide (NOx) from an exhaust gas associated with a diesel engine 10 of a motor vehicle. As is shown, a probe S1, e.g., a zeolite-based ammonia sensor, is positioned within an exhaust gas pipe 102 of the system 100 downstream of a selective catalytic reduction (SCR) catalyst. An ammonia sensor interface unit 106 is coupled to the probe S1 and to a dosing controller 108. The ammonia sensor interface unit 106 provides an indication, which is based on the impedance of the sensor S1, to the dosing controller 108 as to the level of the ammonia in an exhaust gas stream associated with diesel engine 10.

When ammonia breakthrough is detected in the exhaust gas stream, the dosing controller 108 may command a treatment unit 104 to decrease the amount of a urea solution provided into the exhaust stream carried by the exhaust pipe 102. In this manner, the system 100 performs selective catalytic reduction (SCR) to remove nitrogen oxide (NOx) from the exhaust stream of the diesel engine 10, while monitoring for ammonia breakthrough.

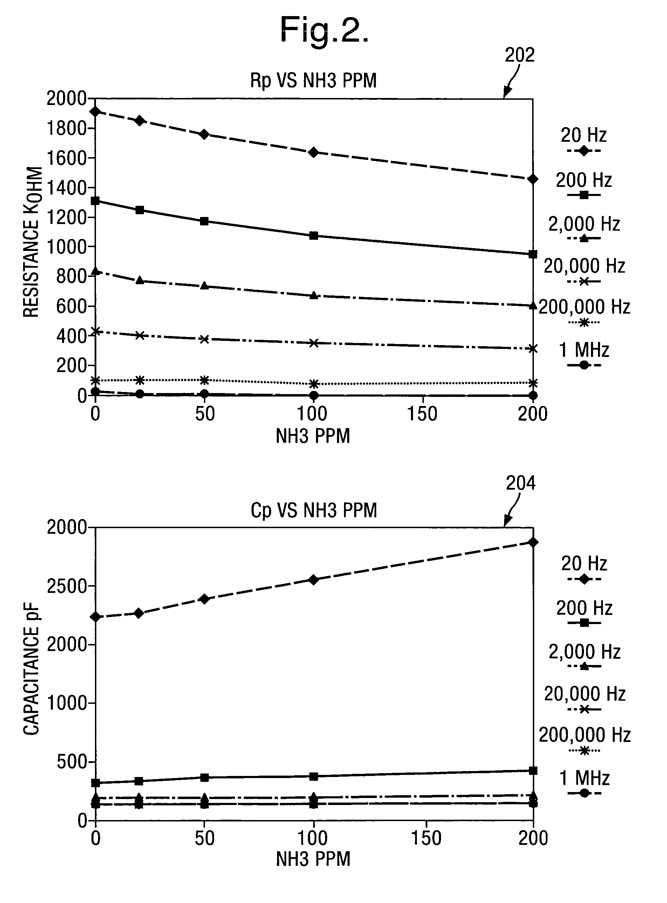

With reference to Fig. 2, graphs 202 and 204 show a number of impedance curves of an exemplary zeolite-based ammonia sensor. Each of the graphs 202 and 204 include impedance curves that show that both of the real and imaginary parts of the impedance change with frequency and the concentration of NH3 in the sample gas being measured. This frequency dependence of both of these components points out the need to correctly select and control the actual times of the two waveform measurement samples to accurately calculate the real and imaginary parts of the sensor impedance and relate it to the desired indication of NH3 concentration.

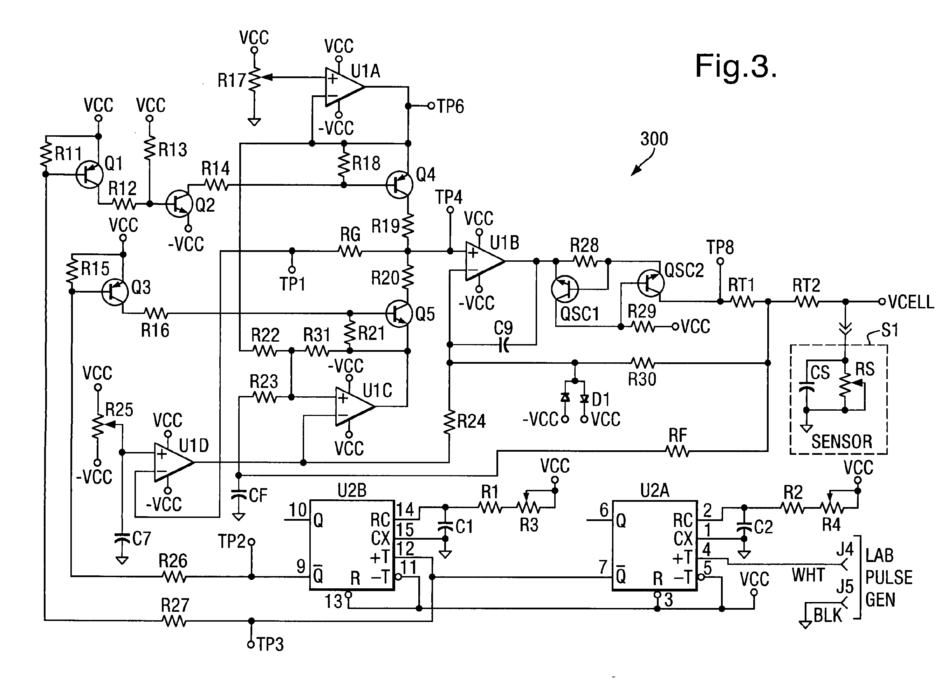

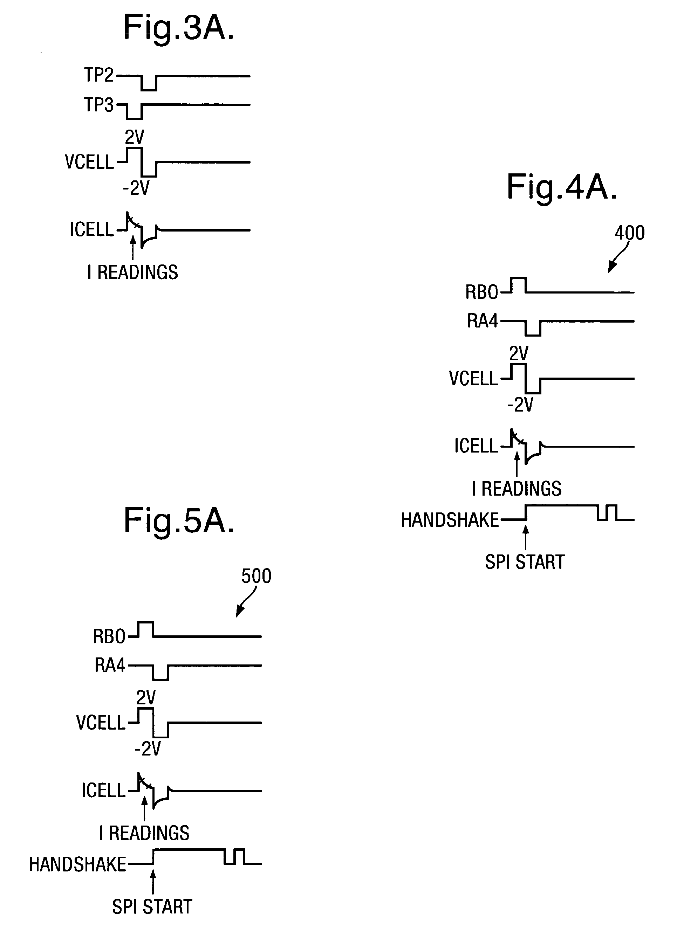

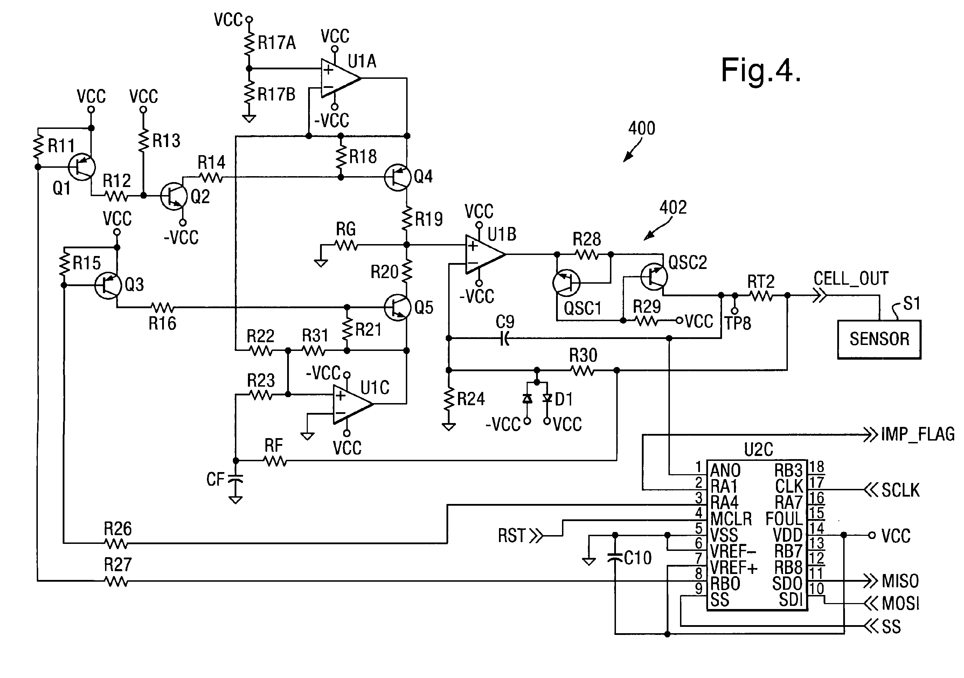

With reference to Figs. 3 and 4, an interface circuit 300 that may be utilized to provide first and second electrical pulses to facilitate determination of a sensor impedance and exemplary signals at various locations in the circuit 300, respectively, are shown. In this embodiment, the first and second electrical pulses have opposite polarity and approximately equal energy, e.g., energies within +/-5 percent of each other. As is shown in Fig. 3, dual monostable multivibrators U2A and U2B are utilized to provide the first electrical pulse and the second electrical pulse, respectively. A pulse generator (not shown) is coupled to and provides a trigger on a trigger input +T of the multivibrator U2A. An output (Q) of the multivibrator U2A is coupled to a trigger input +T of the multivibrator U2B. A resistor R2 and a potentiometer R4, in conjunction with a capacitor C2, are utilized to set the pulse width associated with the multivibrator U2A. A resistor R1 and a potentiometer R3, in conjunction with a capacitor C1, are utilized to set the pulse width associated with the multivibrator U2B.

It should be appreciated that the pulse widths are generally sensor type specific. In general, if one wants to determine a complex sensor impedance it is desirable to take two sensor current readings. In this case, a first current reading is taken while the sensor current is slewing and a second current reading is taken when the sensor current has approximately reached a steady-state, e.g., within +/-5 percent of a steady-state value. At any rate, the first electrical pulse may be terminated upon taking the second current reading. It should also be appreciated that if the resistive component of the impedance is all that is desired, only one current reading, taken when the sensor current is approximately at a steady-state value, is required. In either situation, a single pulse may be utilized to determine a sensor impedance, depending upon a sensor type. That is, a single pulse may be used to determine the impedance of a sensor that recovers relatively quickly from a charge imbalance.

Responsive to the pulse generator, a signal TP3 is provided at the output (Q) of the multivibrator U2A and a signal TP2 is provided at the output of the multivibrator U2B. A signal VCELL shows an exemplary signal provided to a simulated version of sensor S1, which is represented by a potentiometer RS in parallel with a capacitor CS. A signal ICELL represents the current at test point TP8, within the feedback loop of the operational amplifier U1B.

The interface circuit 300 may be set up in a current mode or a voltage mode. In the current mode, a resistor RT1 is provided inside the loop associated with the operational amplifier U1B and a resistor RT2 is replaced with a short. In the voltage mode, the resistor RT1 is replaced with a short and the resistor RT2 is provided outside the loop of the amplifier U1B. As the amplifier U1B is being operated in a closed loop configuration, the voltage outside the loop is a known value, as determined by the components associated with the amplifier U1B. The current flowing through the resistor RT1 can then be determined by measuring the voltage at the test point TP8, as the value of the resistor RT1 is also known.

As mentioned above, the reactive and resistive components of the sensor can then be calculated when two current readings are taken. For example, if the components associated with amplifier U1B are set such that the output of the amplifier U1B goes to one volt, the voltage at the test point TP8 will be one IR (where I is the sensor current and R is the resistance of the resistor RT1) drop higher than the output voltage, which allows the sensor current to be readily calculated.

The transistors Q1, Q2 and Q3, and their associated components, perform a level shifting function to allow the symmetrical driving of the transistors Q4 and Q5. As is shown, the transistor Q4 is a PNP transistor and the transistor Q5 is an NPN transistor and these transistors form switches which pull-up and pull-down a voltage that is provided at test point TP4. It should be appreciated that the pull-up and pull-down voltages are mirror images of each other and are set by the operational amplifier U1A and its associated components. The amplifier U1A is configured as a voltage follower to generate a DC voltage at test point TP6. An operational amplifier U1D and its associated components may be provided to vary a low side of resistor RG from ground (at test point TP1).

The voltage at test point TP6 is divided in half during the pull-up portion of the waveform and the output of the operational amplifier U1C is divided in half on the pull-down portion of the waveform. As the voltage is divided in half, the next stage, which consists of operational amplifier U1B and its associated components, is set for a gain of two. Accordingly, the transistors U1B and U1C are inside the loop and are therefore short circuit protected. A diode package D1 is also included to provide transient switching protection. An RC filter, made up of resistor RF and capacitor CF, provides output feedback to an input of the amplifier U1C and may be implemented to achieve charge balance, if desired. Transistors QSC1 and QSC2 and their associated components provide short circuit protection for the output of the amplifier U1B.

With reference to Fig. 4, an interface circuit 400 that includes a microcontroller U2C and pulse driver circuit 402 is depicted. Fig. 4A depicts exemplary waveforms associated with the circuit 400. The circuit 400 functions in a similar manner to the circuit 300 of Fig. 3, with the exception that the monostable multivibrators U2A and U2B are replaced with the microcontroller U2C and the microcontroller U2C monitors the voltage at the test point TP8 to determine a sensor current, as the circuit 400 is configured for the current mode. Another difference between the circuit 400 of Fig. 4 and the circuit 300 of Fig. 3 is that the operational amplifier U1D and its associated components have been removed and the low side of the resistor RG has been connected directly to ground.

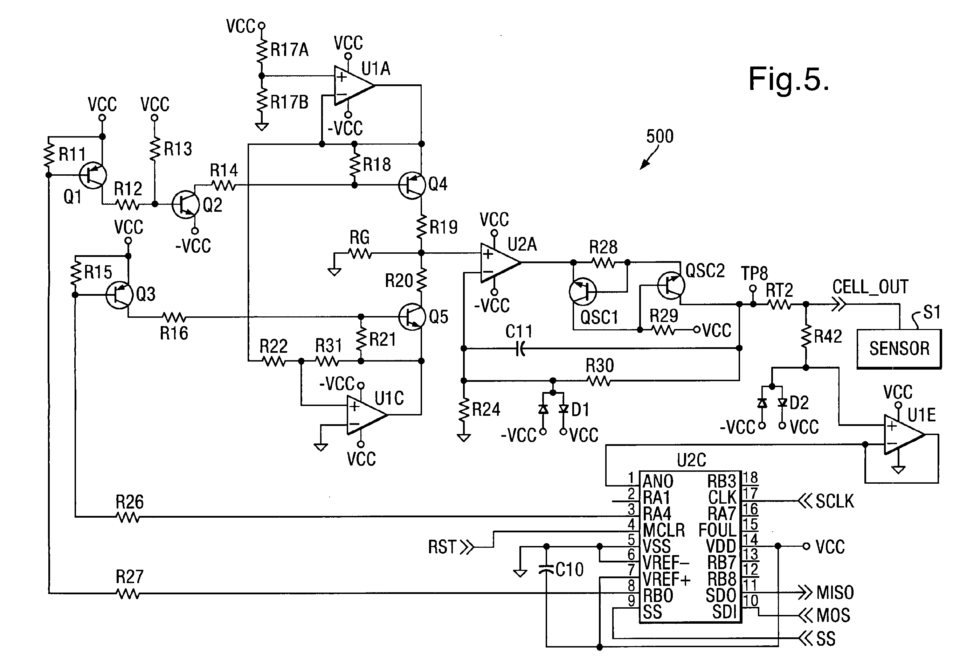

Fig. 5 depicts an interface circuit 500 that is similar to the circuit 400, with the exception that the circuit 500 operates in the voltage mode, i.e., a voltage across the sensor is measured to determine a sensor current. Fig. 5A shows exemplary signals associated with the circuit 500. As is shown in Fig. 5, a non-inverting input of a unity gain operational amplifier U1E is coupled through a 10K ohm resistor to an input of the sensor S1. An output of the amplifier U1E is coupled to an input of the microcontroller U2C, such that the microcontroller U2C can determine the voltage across the sensor S1. It is also contemplated that a matched pair of pull-up and pull-down constant current sources may be utilized to determine the impedance of a sensor.

Accordingly, a method and system have been described herein that advantageously provide a less expensive, less error prone technique for determining the impedance of a sensor, e.g., a zeolite-based ammonia sensor, that may experience ion migration.

The above description is considered that of the preferred embodiments only. Modifications of the invention will occur to those skilled in the art and to those who make or use the invention. Therefore, it is understood that the embodiments shown in the drawings and described above are merely for illustrative purposes and not intended to limit the scope of the invention, which is defined by the following claims as interpreted according to the principles of patent law, including the doctrine of equivalents.

## Claims

1. A method for measuring the impedance of a sensor (S1) subject to ion migration, comprising the steps of:
applying a first electrical pulse (VCELL) to an input of a sensor (S1), wherein an impedance of the sensor (S1) varies according to a first gas concentration in a gas stream;
applying a second electrical pulse (VCELL) to the input of the sensor (S1), wherein the energy of the first and second electrical pulses (VCELL) is approximately the same and the first and second electrical pulses (VCELL) have opposite polarity;
determining a sensor load current (ICELL) during at least one of the first and second electrical pulses (VCELL) to provide a first sensor current (ICELL);
determining the sensor load current (ICELL) during the same one of the first and second electrical pulses (VCELL) to provide a second sensor current (ICELL); and
determining at least one component of the impedance of the sensor (S1) based upon the first and second sensor currents (ICELL).

2. The method of claim 1, wherein the first sensor current (ICELL) is determined during the first electrical pulse (VCELL) at a first time and the second sensor current (ICELL) is determined during the first electrical pulse (VCELL) at a second time that occurs after the first time.

3. The method of claim 1, wherein the first sensor current (ICELL) is determined during the first electrical pulse (VCELL) while the sensor load current (ICELL) is slewing and the second sensor current (ICELL) is determined during the first electrical pulse (VCELL) when the sensor load current (ICELL) has approximately reached a steady-state value.

4. The method of claim 1, wherein the gas stream is an exhaust gas stream associated with a diesel engine (10).

5. The method of claim 1, wherein the at least one component of the impedance is a resistive component.

6. The method of claim 1, wherein the at least one component of the impedance includes a reactive component and a resistive component.

7. The method of claim 1, wherein the sensor (S1) is a zeolite-based sensor.

8. The method of claim 1, wherein the first gas concentration is an ammonia concentration and the gas stream is an exhaust gas stream associated with a diesel engine (10).

9. A method for measuring the impedance of a sensor (S1) subject to ion migration, comprising the steps of:
applying a first electrical pulse (VCELL) to an input of a sensor (S1), wherein an impedance of the sensor (S1) varies according to a first gas concentration in a gas stream;
determining a sensor load current (ICELL) during the first electrical pulse (VCELL) to provide a first sensor current (ICELL); and
determining at least one component of the impedance of the sensor (S1) based upon the first sensor current (ICELL).

10. The method of claim 9, further comprising the steps of:
applying a second electrical pulse (VCELL) to the input of the sensor (S1), wherein the energy of the first and second electrical pulses (VCELL) is approximately the same and the first and second electrical pulses (VCELL) have opposite polarity;
determining the sensor load current (ICELL) during the first electrical pulse (VCELL) to provide a second sensor current (ICELL); and
determining at least one component of the impedance of the sensor (S1) based upon the first and second sensor currents (ICELL).

11. The method of claim 10, wherein the first sensor current (ICELL) is determined during the first electrical pulse (VCELL) at a first time and the second sensor current (ICELL) is determined during the first electrical pulse (VCELL) at a second time that occurs after the first time.

12. The method of claim 10, wherein the first sensor current (ICELL) is determined during the first electrical pulse (VCELL) while the sensor load current (ICELL) is slewing and the second sensor current (ICELL) is determined during the first electrical pulse (VCELL) when the sensor load current (ICELL) has approximately reached a steady-state value.

13. The method of claim 10, wherein the gas stream is an exhaust gas stream associated with a diesel engine (10).

14. The method of claim 10, wherein the at least one component of the impedance is a resistive component.

15. The method of claim 10, wherein the at least one component of the impedance includes a reactive component and a resistive component.

16. The method of claim 10, wherein the sensor (S1) is a zeolite-based sensor.

17. The method of claim 10, wherein the first gas concentration is an ammonia concentration and the gas stream is an exhaust gas stream associated with a diesel engine (10).

18. A system (400) for measuring the impedance of a sensor (S1) subject to ion migration, comprising:
a pulse driver circuit (402);
a processor (U2C) coupled to the pulse driver circuit (402), the processor (U2C) executing code that instructs the processor (U2C) to perform the steps of:
causing the pulse driver circuit (402) to provide a first electrical pulse (VCELL) to an input of a sensor (S1), wherein an impedance of the sensor (S1) varies according to a first gas concentration in a gas stream;
causing the pulse driver circuit (402) to provide a second electrical pulse (VCELL) to the input of the sensor (S1), wherein the energy of the first and second electrical pulses (VCELL) is approximately the same and the first and second electrical pulses (VCELL) have opposite polarity;
determining a sensor load current (ICELL) during at least one of the first and second electrical pulses (VCELL) to provide a first sensor current (ICELL);
determining the sensor load current (ICELL) during the same one of the first and second electrical pulses (VCELL) to provide a second sensor current (ICELL); and
determining at least one component of the impedance of the sensor (S1) based upon the first and second sensor currents (ICELL).

19. The system (400) of claim 18, wherein the first sensor current (ICELL) is determined during the first electrical pulse (VCELL) at a first time and the second sensor current (ICELL) is determined during the first electrical pulse (VCELL) at a second time that occurs after the first time.

20. The system (400) of claim 18, wherein the first sensor current (ICELL) is determined during the first electrical pulse (VCELL) while the sensor load current (ICELL) is slewing and the second sensor current (ICELL) is determined during the first electrical pulse (VCELL) when the sensor load current (ICELL) has approximately reached a steady-state value.

21. The system of claim 18, wherein the sensor (S1) is a zeolite-based sensor and the first gas concentration is an ammonia concentration, and wherein the gas stream is an exhaust gas stream associated with a diesel engine (10).
